# EUROPÄISCHE PATENTSCHRIFT

(11) **EP 3 738 634 B1**
(45) Veröffentlichungstag und Bekanntmachung des Hinweises auf die Patenterteilung: **02.10.2024**
(21) Anmeldenummer: 20000177.4
(22) Anmeldetag: 07.05.2020
(51) Int. Cl.: A61M 16/00, F04D 29/16, F04D 29/28, F04D 29/30

(54) **ATEMTHERAPIEGERÄT UND LÜFTERRAD FÜR EIN ATEMTHERAPIEGERÄT**
BREATHING THERAPY DEVICE AND FAN FOR BREATHING THERAPY DEVICE
APPAREIL DE THÉRAPIE RESPIRATOIRE ET ROUE DE VENTILATEUR POUR UN APPAREIL DE THÉRAPIE RESPIRATOIRE

(30) Priorität: 16.05.2019 DE 102019112864
(43) Veröffentlichungstag der Anmeldung: 18.11.2020
(73) Patentinhaber: Löwenstein Medical Technology S.A., 2557 Luxembourg (LU)
(72) Erfinder: Gerlach, Angela, 22607 Hamburg (DE)
(74) Vertreter: Marx, Thomas

(56) Entgegenhaltungen:
- KR-A- 20050 119 071
- US-A1- 2007 116 576
- US-A1- 2015 328 418
- US-A1- 2020 101 245

## Beschreibung

Die vorliegende Erfindung betrifft ein Atemtherapiegerät mit wenigstens einer Lüftereinrichtung zum Erzeugen einer Atemluftströmung für die Durchführung einer Atemtherapie. Die Lüftereinrichtung umfasst wenigstens ein drehbares Lüfterrad mit einer Mehrzahl von Schaufelelementen.

Solche Geräte werden beispielsweise zur Beatmung bzw. Atmungsunterstützung oder zur Hustenunterstützung eingesetzt. Damit die Behandlung bzw. Therapie nicht als störend empfunden wird, sollten die Betriebsgeräusche des Lüfters möglichst gering sein. US 2015/0328418 A1 offenbart ein Atemtherapiegerät mit einem zweigeteilten Gehäuse. Für eine gezielte Atemtherapie ist es zudem entscheidend, dass möglichst zügige und kurzfristige Drehzahlanpassungen des Lüfterrades erfolgen können.

Demgegenüber ist es die Aufgabe der vorliegenden Erfindung, ein Atemtherapiegerät zur Verfügung zu stellen, welches die zuvor genannten Anforderungen möglichst vorteilhaft erfüllen kann.

Diese Aufgabe wird mit einem Atemtherapiegerät des Anspruchs 1 sowie mit einem Lüfterrad gemäß Anspruch 17 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße Atemtherapiegerät umfasst wenigstens eine Lüftereinrichtung zum Erzeugen einer Atemluftströmung für die Durchführung einer Atemtherapie. Die Lüftereinrichtung umfasst wenigstens ein drehbares Lüfterrad. Das Lüfterrad umfasst eine Mehrzahl von Schaufelelementen. Dabei sind die Schaufelelemente wenigstens teilweise mit jeweils wenigstens einem Winglet ausgestattet. Dabei verläuft das Winglet wenigstens abschnittsweise an wenigstens einer axialen Längsseite des Schaufelelements. Dabei ist das Lüfterrad an einer axialen Seite mit einer Tragscheibe ausgestattet, wobei die Schaufelelemente an der Tragscheibe und an einer Nabe befestigt sind.

Das erfindungsgemäße Atemtherapiegerät bietet viele Vorteile. Einen erheblichen Vorteil bieten die mit den Winglets ausgestatteten Schaufelelemente. Dadurch ist das Lüfterrad dazu geeignet und ausgebildet, die Austauschströmung von der Druckseite zur Saugseite der Schaufelelemente sowie die Austauschströmung aus dem Seitenraum zu den Schaufelkanälen besonders wirksam und zuverlässig zu unterbinden. Ein besonderer Vorteil der Erfindung ist, dass die Unterbindung der Austauschströmung mit einer besonders geringen Masse an Bauteilen und zudem konstruktiv unaufwendig verwirklicht wird. So ist das Lüfterrad der Erfindung erheblich leichter als ein Lüfterrad mit einer Deckscheibe oder anderen Scheiben zur Reduzierung von Austauschströmungen. Dadurch weist das Lüfterrad eine besonders geringe Trägheit auf, sodass Drehzahlanpassungen besonders zügig und mit einem geringen Leistungsaufwand möglich sind.

Zudem kann die Lüftereinrichtung der Erfindung im Vergleich zu herkömmlichen Lüftern für Atemtherapiegeräte bei gleichem Druck mit geringeren Drehzahlen betrieben werden. Das bringt wiederum erheblich geringere Betriebsgeräusche mit sich, sodass eine besonders leise Therapie erfolgen kann. Zudem hat sich herausgestellt, dass mit der Erfindung auch ein erheblich verbesserter Wirkungsgrad für die Erzeugung der Atemluftströmung erreicht wird. Die Erfindung bietet daher erhebliche Vorteile gegenüber anderen Ansätzen zur Verhinderung von Austauschverlusten bzw. Austauschströmungen.

Vorzugsweise ist das Winglet wenigstens in Richtung einer Saugseite des Schaufelelements gerichtet. Dabei ist die Saugseite insbesondere die einer Drehrichtung des Lüfterrades abgewandte Seite des Schaufelelements. Insbesondere ist eine Druckseite die der Drehrichtung des Lüfterrades zugewandte Seite des Schaufelelements. Eine solche Anordnung der Winglets ist besonders vorteilhaft und bietet beispielsweise sowohl eine Druck- als auch eine Wirkungsgradsteigerung im Vergleich zu einem Lüfterrad ohne Winglets.

Bevorzugt ist auch, dass das Winglet in Richtung einer Druckseite und einer Saugseite des Schaufelelements gerichtet ist. Eine solche Ausgestaltung ist besonders vorteilhaft zur Erzielung einer Drucksteigerung einsetzbar. Möglich ist auch, dass das Winglet nur in Richtung einer Druckseite des Schaufelelements gerichtet ist.

In einer besonders vorteilhaften Ausgestaltung ist das Winglet derart an der Längsseite des Schaufelelements angeordnet, dass sich das Winglet in Richtung einer Saugseite in gleicher Ausdehnung wie in Richtung einer Druckseite des Schaufelelements erstreckt. Insbesondere verläuft die Längsseite des Schaufelelements mittig zum Winglet bzw. zentriert über das Winglet. Insbesondere ist das Winglet mittig bzw. zentriert auf dem Schaufelelement angeordnet.

Es ist vorteilhaft und bevorzugt, dass das Winglet eine Ausdehnung von 1° bis 20° des Umfangs des Lüfterrades aufweist. Besonders bevorzugt weist das Winglet.eine Ausdehnung von 5° bis 15° des Umfangs des Lüfterrades auf. Der Umfang des Lüfterrades beträgt insbesondere 360°. Die Ausdehnung des Winglets entspricht insbesondere einem Kreissektor, - auch als Kreisausschnitt bezeichnet. Die Ausdehnung des Winglets beträgt insbesondere 10° +/-3° und vorzugsweise 10° +/-2° und besonders bevorzugt 10° +/-1° des Umfangs des Lüfterrades. Die Ausdehnung des Winglets kann auch 10° +/-0,5° oder 10° des Umfangs des Lüfterrades betragen. Vorzugsweise beträgt die Ausdehnung der Winglets weniger als 21° und besonders bevorzugt weniger als 20° des Umfangs des Lüfterrades. Möglich ist auch, dass das Winglet eine Ausdehnung bis zu 25° oder mehr des Umfangs des Lüfterrades aufweist. Solche Dimensionierungen der Winglets haben sich als besonders vorteilhaft erwiesen. Diese Angaben betreffen insbesondere die Ausdehnung des Winglets über dessen gesamte Länge oder an dessen schmalster oder breitester Stelle oder an dessen Mitte oder Ende.

Insbesondere nimmt eine Ausdehnung des Winglets von radial innen des Lüfterrades nach radial außen des Lüfterrades zu. Insbesondere ist das Winglet radial innen schmaler ausgebildet als radial außen. Es ist möglich, dass wenigstens eine andere charakteristische Geometrieeigenschaft des Winglets von radial innen nach radial außen angepasst bzw. verändert wird.

Erfindungsgemäß ist das Lüfterrad an einer axialen Seite, mit wenigstens einer Scheibe ausgestattet. Die Scheibe ist als Tragscheibe zur Befestigung der Schäufelelemente geeignet und ausgebildet. Es ist ein besonderer Vorteil der Erfindung, dass wenigstens an der Seite mit den Winglets eine solche Scheibe eingespart wird. Die Winglets bieten dabei einen besonders vorteilhaften Ersatz für eine solche Scheibe, da diese ein erheblich geringeres Gewicht und einen leiseren Betrieb sowie auch verbesserte Strömungseigenschaften ermöglichen.

Es kann vorgesehen sein, dass trotz der Winglets Scheiben an beiden axialen Seiten des Lüfterrades vorgesehen sind, beispielsweise zur Befestigung der Schaufelelemente oder auch mit einer strömungstechnischen Funktion.

Die Scheibe kann fest mit dem Lüfterrad verbunden sein. Möglich ist auch, dass die Scheibe separat zum Lüfterrad ausgeführt ist bzw. ein vom Lüfterrad getrenntes Bauteil darstellt.

Insbesondere ist die Tragscheibe fest mit dem Lüfterrad verbunden und/oder die Deckscheibe separat zum Lüfterrad ausgebildet.

Die Scheibe, insbesondere die Deckscheibe und/oder die Tragscheibe, weist insbesondere eine radial innen liegende Ansaugöffnung und/oder Ausströmöffnung auf. Es ist möglich, dass die Schaufelelemente über den Umfang der Deckscheibe hinausragen. Die Deckscheibe ist insbesondere nicht mit den Schaufelelementen verbunden. Die Deckscheibe dient insbesondere nicht zur Befestigung der Schaufelelemente.

Die Tragscheibe, trägt die Schaufelelemente. Die Tragscheibe ist fest mit den Schaufelelementen verbunden und vorzugsweise einstückig mit den Schaufelelementen ausgebildet. Insbesondere weist die Tragscheibe eine zentrale Ausnehmung auf, beispielsweise als Teil einer Nabe zum Durchführen einer Antriebswelle. Die Schaufelelemente können über den Umfang der Tragscheibe hinausragen. Die Scheibe kann geschlossen ausgebildet sein.

In einer vorteilhaften und bevorzugten Ausgestaltung ist die Scheibe wenigstens, insbesondere nur, an derjenigen axialen Seite des Lüfterrades angeordnet, welche einer mit den Winglets ausgestatteten axialen Seite des Lüfterrades gegenüberliegt. Somit sind an einer axialen Seite des Lüfterrades die Winglets und an der gegenüberliegenden axialen Seite die Scheibe angeordnet. Eine solche Anordnung von Scheibe und Winglets bietet besonders viele strömungstechnische Vorteile. Bevorzugt ist dabei die Tragscheibe an derjenigen axialen Seite des Lüfterrades angeordnet, welche der mit den Winglets ausgestatteten axialen Seite gegenüberliegt. In einer solchen Ausgestaltung kann das Lüfterrad als ein Tragscheibenlüfterrad ohne Deckscheibe und mit Winglets bezeichnet werden. Möglich ist aber auch, dass die Deckscheibe derartig angeordnet ist. Insbesondere sind die Schaufelelemente mit einer axialen Längsseite an der Scheibe angeordnet bzw. befestigt, welche nicht mit den Winglets ausgestattet ist.

Die Schaufelelemente sind vorzugsweise innerhalb eines Umfangs der Scheibe angeordnet. Insbesondere stehen die Schaufelelemente nicht über den Umfang der Scheibe hinaus. Insbesondere erstrecken sich auch die Winglets innerhalb eines Umfangs der Scheibe und/oder stehen nicht über den Umfang der Scheibe hinaus. Möglich ist aber auch, dass die Schaufelelemente und/oder die Winglets über den Umfang der Scheibe hinausragen.

In einer besonders vorteilhaften Ausgestaltung sind die Schaufelelemente wenigstens teilweise und vorzugsweise vollständig gerade ausgebildet. Das ermöglicht eine besonders unaufwendige und wirtschaftliche Herstellung des Lüfterrades, beispielsweise mittels eines Spritzgussverfahrens oder dergleichen. Insbesondere sind die Schaufelelemente eben bzw. planar ausgebildet. Insbesondere stehen die Flächen der Schaufelelemente rechtwinkelig auf der Hauptebene des Lüfterrades. Beispielsweise sind sogenannte 90°-Schaufeln vorgesehen. Es können auch 90°-Schaufeln +/- 15° vorgesehen sein. Möglich und bevorzugt ist auch, dass die Schaufelelemente gekrümmt ausgebildet sind. Die Schaufelelemente können über ihre Längsausdehnung verdreht bzw. helixartig ausgebildet sein. Die Schaufelelemente können auch gebogen sein, ohne dabei um ihre Längsachse verdreht zu sein.

Insbesondere sind die Schaufelelemente des Lüfterrades alle identisch ausgebildet. Möglich ist aber auch, dass die Schaufelelemente des Lüfterrades unterschiedlich ausgebildet sind. Insbesondere sind die Schaufelelemente dabei wahlweise, beispielsweise abwechselnd, mit den zuvor genannten Merkmalen ausgebildet.

Vorzugsweise ist das Lüfterrad durch wenigstens ein Spritzgussverfahren hergestellt bzw. herstellbar. Es können auch andere geeignete Verfahren zur Herstellung vorgesehen sein. Insbesondere sind die Schaufelelemente und/oder die Winglets und/oder die Nabe derartig hergestellt. Das Lüfterrad kann aus Kunststoff oder Metall oder auch einem Verbundwerkstoff gefertigt sein.

Das Lüfterrad ist insbesondere einstückig ausgeführt. Insbesondere sind die Schaufelelemente und die Winglets und insbesondere auch die Nabe einstückig miteinander hergestellt und verbunden. Möglich ist auch, dass das Lüfterrad einstückig mit der Tragscheibe und/oder Deckscheibe verbunden ist. Das Lüfterrad kann auch mehrteilig ausgeführt sein.

Das erfindungsgemäße Lüfterrad ist für ein Atemtherapiegerät vorgesehen, wie es vorzugsweise zuvor beschrieben wurde. Das Lüfterrad umfasst eine Mehrzahl von Schaufelelementen. Dabei sind die Schaufelelemente wenigstens teilweise mit jeweils wenigstens einem an wenigstens einer axialen Längsseite des Schaufelelements wenigstens abschnittsweise verlaufenden Winglet ausgestattet. Dabei ist das Lüfterrad an einer axialen Seite mit einer Tragscheibe ausgestattet, wobei die Schaufelelemente an der Tragscheibe und an einer Nabe befestigt sind.

Dabei ist das Lüfterrad vorzugsweise wie zuvor für das erfindungsgemäße Atemtherapiegerät beschrieben ausgebildet.

Das Lüfterrad ist als ein Impeller ausgeführt sein und von wenigstens einem Impellergehäuse ummantelt sein. Das Gehäuse weist dabei insbesondere wenigstens eine Ansaugöffnung und wenigstens eine Abgabeöffnung auf. Das Lüfterrad ist insbesondere dazu geeignet und ausgebildet, von axial anzusaugen und nach radial auszublasen bzw. zu verdichten. Es können auch andere Anordnungen vorgesehen sein, beispielsweise kann von axial angesaugt und nach axial ausgeblasen werden oder auch von radial angesaugt und nach radial ausgeblasen werden. Das Lüfterrad ist als Radiallüfterrad ausgeführt.

Die Lüftereinrichtung umfasst insbesondere wenigstens eine Antriebseinrichtung und beispielsweise wenigstens einen Elektromotor. Die Lüftereinrichtung ist insbesondere mit wenigstens einer Steuereinrichtung wirkverbunden und über diese ansteuerbar. Beispielsweise sind gezielt bestimmte Drehzahlen für das Lüfterrad einstellbar. Die Lüftereinrichtung umfasst insbesondere wenigstens einen Ansaugbereich und wenigstens einen Abgabebereich. Es ist möglich, dass der Ansaugbereich und/oder der Abgabebereich durch wenigstens eine Scheibe und/oder wenigstens ein Gehäuse oder dergleichen bereitgestellt werden. Erfindungsgemäß wird unter einem Winglet eine flächige und entlang einer Längsseite des Schaufelelements angeordnete strömungstechnisch wirksame Struktur verstanden. Insbesondere sind die Winglets der einzelnen Schaufelelemente separat zueinander ausgebildet und weisen insbesondere keine Verbindung zueinander auf. Insbesondere sind die Winglets nicht derart miteinander verbunden, dass sich eine geschlossene und beispielsweise scheibenartige Struktur ergibt. Die Winglets sind insbesondere als axiale Abkantungen bzw. Abkantungen an einer axialen Längsseite der Schaufelelemente ausgebildet. Dabei können die Abkantungen so ausgebildet sein, dass sich ein L-förmiger Querschnitt des Schaufelelements zusammen mit dem Winglet ergibt. Die Abkantungen können auch beidseitig ausgeführt sein, sodass sich ein T-förmiger Querschnitt ergibt. Erfindungsgemäß muss ein Winglet nicht in einem Winkel von 90° zur Schaufel angeordnet sein; die Winglets können auch <90° oder >90° zur Schaufel angeordnet sein.

Die Abkantungen können abgerundet sein. Insbesondere sind alle Schaufelelemente mit jeweils wenigstens einem und insbesondere mit nur einem Winglet ausgestattet. Möglich ist auch, dass jedes zweite oder dritte oder vierte Schaufelelement mit jeweils wenigstens einem Winglet ausgestattet ist. Es ist möglich, dass eine überwiegende Mehrzahl der Schaufelelemente mit jeweils wenigstens einem Winglet ausgestattet ist.

Das Winglet verläuft insbesondere entlang der gesamten axialen Längsseite des Schaufelelements. Es ist möglich, dass das Winglet über wenigstens die Hälfte und insbesondere über wenigstens 75 % und vorzugsweise über wenigstens 85 % und besonders bevorzugt über wenigstens 90 % der Länge der axialen Längsseite des Schaufelelements verläuft. Das Winglet kann auch über wenigstens 95 % oder wenigstens 98 % der Länge der axialen Längsseite des Schaufelelements verlaufen. Das Winglet ist insbesondere durchgehend ausgebildet. Das Winglet kann auch wenigstens eine Unterbrechung aufweisen.

Das Winglet ist insbesondere an nur einer axialen Längsseite des Schaufelelements- angeordnet. Möglich- ist auch, dass an beiden axialen Längsseiten des Schaufelelements jeweils wenigstens ein Winglet wenigstens abschnittsweise verläuft.

Die Winglets sind insbesondere identisch ausgebildet. Die Winglets sind insbesondere dann identisch ausgebildet, wenn die Winglets an identisch ausgebildeten Schaufelelementen angeordnet sind. Die Schaufelelemente können auch verschieden ausgestaltete Winglets aufweisen.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine rein schematische Darstellung eines erfindungsgemäßen Atemtherapiegeräts in einer perspektivischen Ansicht;
- Fig. 2: eine rein schematische Darstellung eines erfindungsgemäßen Lüfterrades in einer perspektivischen Ansicht;
- Fug. 3: das Lüfterrad der Fig. 2 in einer Seitenansicht;
- Fig. 4: das Lüfterrad der Fig. 2 in einer Draufsicht;
- Fig. 5: ein weiteres erfindungsgemäßes Lüfterrad in einer perspektivischen Ansicht;
- Fug. 6: das Lüfterrad der Fig. 5 in einer Seitenansicht;
- Fig. 7: das Lüfterrad der Fig. 5 in einer Draufsicht;
- Fig. 8: ein weiteres erfindungsgemäßes Lüfterrad in einer perspektivischen Ansicht;
- Fug. 9: das Lüfterrad der Fig. 8 in einer Seitenansicht;
- Fig. 10: das Lüfterrad der Fig. 8 in einer Draufsicht;
- Fig. 11: ein weiteres erfindungsgemäßes Lüfterrad in einer Draufsicht;
- Fig. 12: ein stark schematisiertes Schaubild mit Kennlinien für Lüfterräder; und
- Fug. 13: ein weiteres stark schematisiertes Schaubild mit Kennlinien für Lüfterräder.

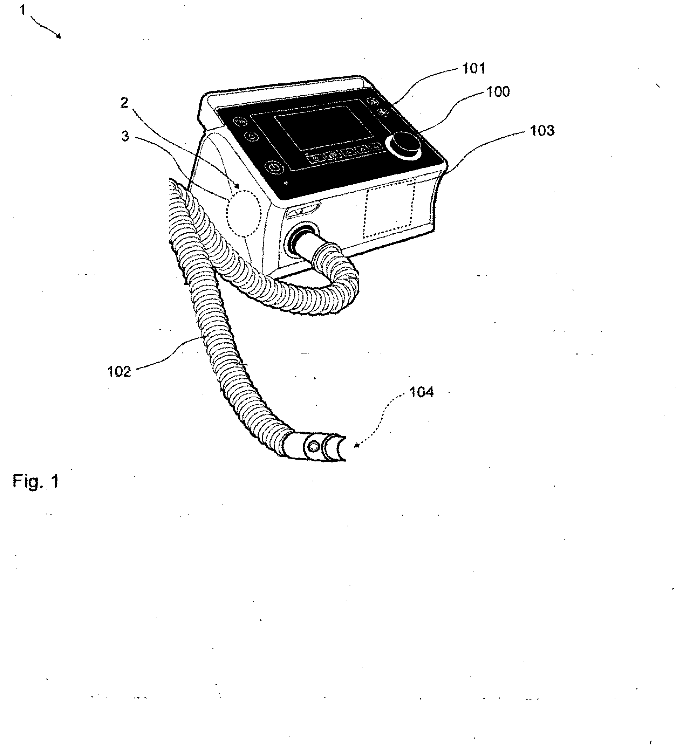

Die Figur 1 zeigt ein erfindungsgemäßes Atemtherapiegerät 1, welches beispielsweise ein Beatmungsgerät oder ein Hustengerät ist. Das Gerät 1 ist mit einer hier nicht sichtbar im Geräteinneren untergebrachten Lüftereinrichtung 2 ausgestattet, mit der die Atemluftströmung für die Atemtherapie erzeugt wird. Die Lüftereinrichtung 2 ist mit einem erfindungsgemäßen Lüfterrad 3 ausgestattet. Zum Drehen des Lüfterrades 3 weist die Lüftereinrichtung 2 einen elektrischen Antrieb auf.

Die Lüftereinrichtung 2 wird über eine nicht sichtbar im Geräteinneren angeordnete Steuereinrichtung 103 angesteuert. Beispielsweise stellt die Steuereinrichtung 103 in Abhängigkeit der Therapievorgaben eine bestimmte Drehzahl des Lüfterrades 3 ein bzw. regelt die Lüfterdrehzahl auf einen Sollwert.

Das Atemtherapiegerät 1 ist hier mit einer Bedieneinrichtung 100 und einer Anzeigeeinrichtung 101 ausgestattet. Dabei erfolgt hier ein Teil der Bedienung über eine berührungsempfindliche Oberfläche der Anzeigeeinrichtung 101. Das Atemtherapiegerät 1 weist eine Schnittstelle zum Koppeln eines Schlauches 102 zur Beatmung bzw. Hustenunterstützung auf. Die mittels der Lüftereinrichtung 2 erzeugte Atemluftströmung wird über die Schlaucheinrichtung 102 dem Patienten zugeführt. An den Schlauch-102 kann eine hier nicht näher dargestellte Patientenschnittstelle und beispielsweise eine Atemmaske angeschlossen werden.

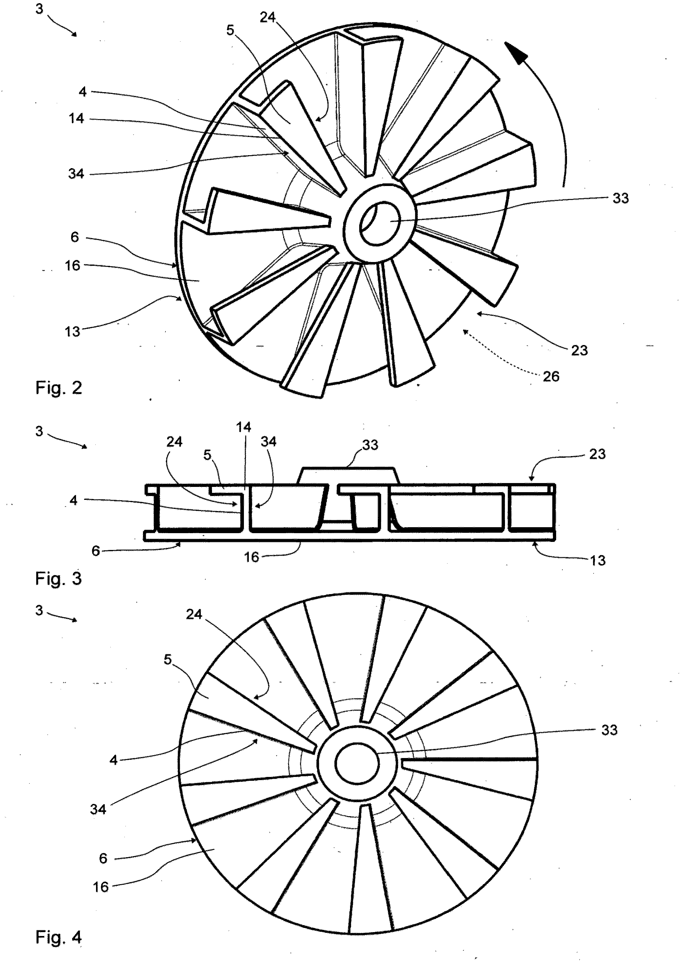

Die Figuren 2 bis 4 zeigen ein erfindungsgemäßes Lüfterrad 3 in verschiedenen Ansichten. Das Lüfterrad 3 ist hier mit einer Mehrzahl von Schaufelelementen 4 und einer als Tragscheibe 16 ausgebildeten Scheibe 6 sowie einer Nabe 33 zur Verbindung mit einer Antriebswelle ausgestattet. Die Schaufelelemente 4 sind hier an der Tragscheibe 16 und vorzugsweise auch an der Nabe 33 befestigt und beispielsweise einstückig mit diesen verbunden.

Die bevorzugte Drehrichtung des Lüfterrades 3 ist hier durch einen Pfeil veranschaulicht. Die Schaufelelemente 4 weisen eine Saugseite 24 und eine Druckseite 34 auf. Für die hier gezeigte Drehrichtung des Lüfterrades 3 ergibt sich die hier gezeigte Ausrichtung von Saugseite 24 und Druckseite 34.

Um die Austauschströmung aus dem Seitenraum sowie von der Druckseite 34 zur Saugseite 24 in Richtung des Seitenraumes bzw. einer axialen Seite wirksam und zuverlässig zu unterbinden, ist das Lüfterrad 3 mit Winglets 5 ausgestattet. Dabei ist hier jedes Schaufelelement 4 mit einem Winglet 5 ausgestattet. Die Winglets 5 verlaufen jeweils entlang einer axialen Längsseite 14 des Schaufelelements 4. In der hier gezeigten Ausgestaltung der Erfindung zeigen die Winglets 5 in Richtung der Saugseite 24 des jeweiligen Schaufelelements 4. Für ein solches Lüfterrad 3 konnte sowohl eine vorteilhafte Drucksteigerung als auch eine erhebliche Wirkungsgradsteigerung beobachtet werden.

Das hier gezeigte Lüfterrad 3 ist beispielsweise so in die Lüftereinrichtung 2 integriert, dass von axial angesaugt und nach radial ausgeblasen wird. Dabei liegt die Ansaugseite an der axialen Seite 23 mit den Winglets 5.

In Fig. 3 ist erkennbar, dass die Materialstärke der Scheibe 6, der Schaufelelemente 4 und der Winglets 5 ähnlich stark gewählt ist. Erfindungsgemäß ist auch vorgesehen die Materialstärke der Scheibe 6, der Schaufelelemente 4 und der Winglets 5 entsprechend der mechanischen Belastungen der einzelnen genannten Bauteile zu optimieren, sodass eine optimale Haltbarkeit zugleich aber eine möglichst geringe Masse des Lüfterrades 3 resultiert. Beispielsweise weisen die Winglets 5 eine geringere Materialstärke auf als die Scheibe 6. Beispielsweise weisen die Winglets 5 eine geringere Materialstärke auf als die Scheibe 6 und als die Schaufelelemete 4. Beispielsweise weisen die Winglets 5 eine geringere Materialstärke auf als die Schaufelelemete 4 und die Schaufelelemete 4 eine geringere Materialstärke als die Scheibe 6.

In der hier gezeigten Ausgestaltung ist das Lüfterrad 3 nur an einer axialen Seite 13 mit der Scheibe 6 ausgestattet. Dabei ist die als Tragscheibe 16 ausgebildete Scheibe 6 an derjenigen axialen Seite 13 des Lüfterrades 3 angeordnet, welche der mit den Winglets 5 ausgestatteten axialen Seite 23 des Lüfterrades 3 gegenüberliegt.

In einer Weiterbildung kann auch eine hier nicht näher dargestellte und als Deckscheibe 26 ausgebildete Scheibe 6 vorgesehen sein. Dabei kann die Deckscheibe 26 zusätzlich zu der Tragscheibe 16 an der axialen Seite 23 angeordnet sein.

Bevorzugt ist die axiale Seite 23 mit den Winglets 5 jedoch ohne Scheibe 6 ausgestattet, da die Winglets 5 die Austauschströmung nicht nur wirksam unterbinden, sondern zugleich auch erheblich weniger Masse mit sich bringen als z. B. eine Deckscheibe 23. Dadurch bietet das hier, gezeigte Lüfterrad 3 besonders gute Strömungseigenschaften und weist zudem eine besonders geringe Trägheit auf, sodass das Lüfterrad 3 besonders zügig beschleunigt werden kann. Durch die Winglets 5 kann somit auf eine Scheibe 6 an der axialen Seite 23 verzichtet werden, ohne dass es zu unerwünschten Austauschströmungen und damit zu ungünstigen Verlusten kommt. Dabei können mit dem erfindungsgemäßen Lüfterrad 3 zugleich erheblich unaufwendiger und schneller Drehzahlanpassungen vorgenommen werden. Ein weiterer Vorteil ist, dass aufgrund der Winglets 5 mehr Druck als bei einem Lüfterrad ohne Winglets erzeugt werden kann, sodass das Lüfterrad 3 zur Erreichung des gleichen Betriebspunktes mit geringerer Drehzahl betrieben werden kann. Daraus ergibt sich ein erheblich leiserer Betrieb des Atemtherapiegeräts 1, sodass die Therapie als komfortabler und angenehmer empfunden wird. Das hier gezeigte Lüfterrad 3 weist gerade Schaufelelemente 4 auf. Möglich sind aber auch andere Ausführungen, beispielsweise mit gekrümmten Schaufelelementen 4. Eine solche Ausführung kann besonders vorteilhaft im Spritzguss hergestellt werden. Das hier gezeigte Lüfterrad 3 ist einstückig hergestellt.

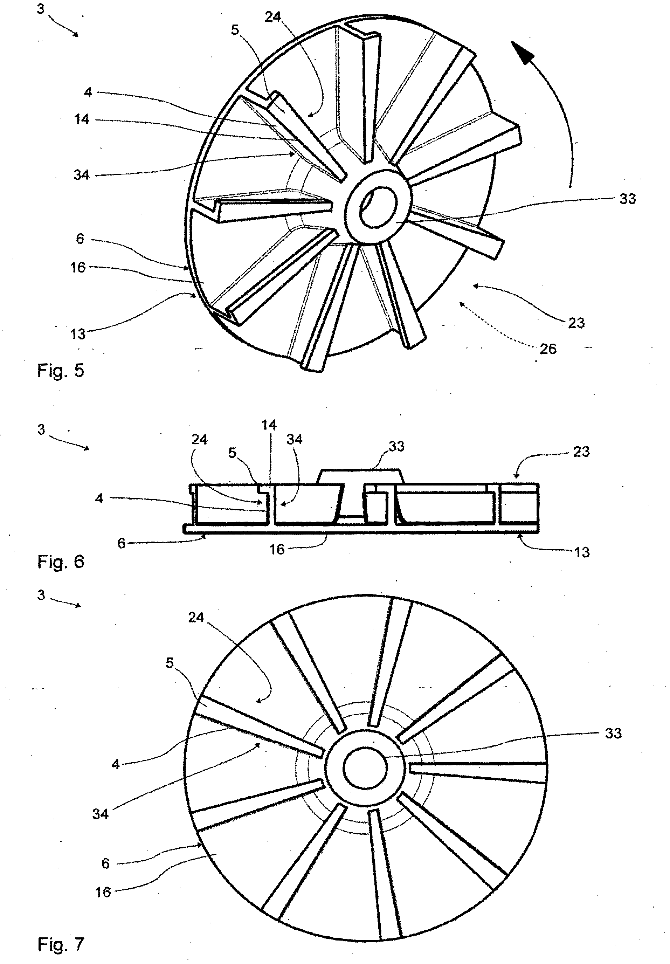

In den Figuren 5 bis 7 ist ein erfindungsgemäßes Lüfterrad 3 gezeigt, welches kürzere Winglets 5 als das zuvor beschriebene Lüfterrad 3 aufweist.

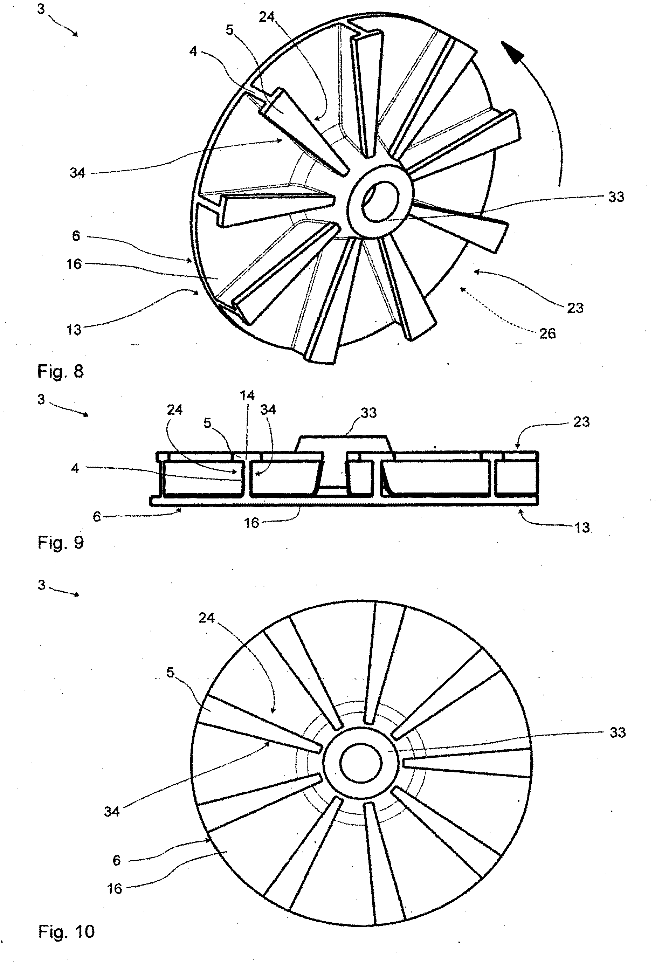

Die Figuren 8 bis 10 zeigen ein erfindungsgemäßes Lüfterrad 3, welches mit Winglets 5 zu beiden Seiten der Schaufelelemente 4 ausgestattet ist. Die Winglets 5 erstrecken, sich somit sowohl in Richtung der Druckseite 34 als auch in Richtung der Saugseite 24 der Schaufelelemente 4. Dabei sind die Winglets 5 4 zentriert auf den Längsseiten 14 der Schaufelelemente 4 angeordnet, sodass sich die Winglets 5 in gleicher Ausdehnung in Richtung der Saugseite 24 wie in Richtung der Druckseite 34 erstrecken. Für ein solches Lüfterrad 3 konnte eine besonders vorteilhafte Drucksteigerung beobachtet werden.

Wenn die Winglets 5 auf beiden Seiten 24, 34 der Schaufelelemente 4 angeordnet sind, werden besonders vorteilhafte Strömungseigenschaften erzielt, wenn- diese entsprechend kurz ausgebildet sind, wie beispielsweise hier gezeigt.

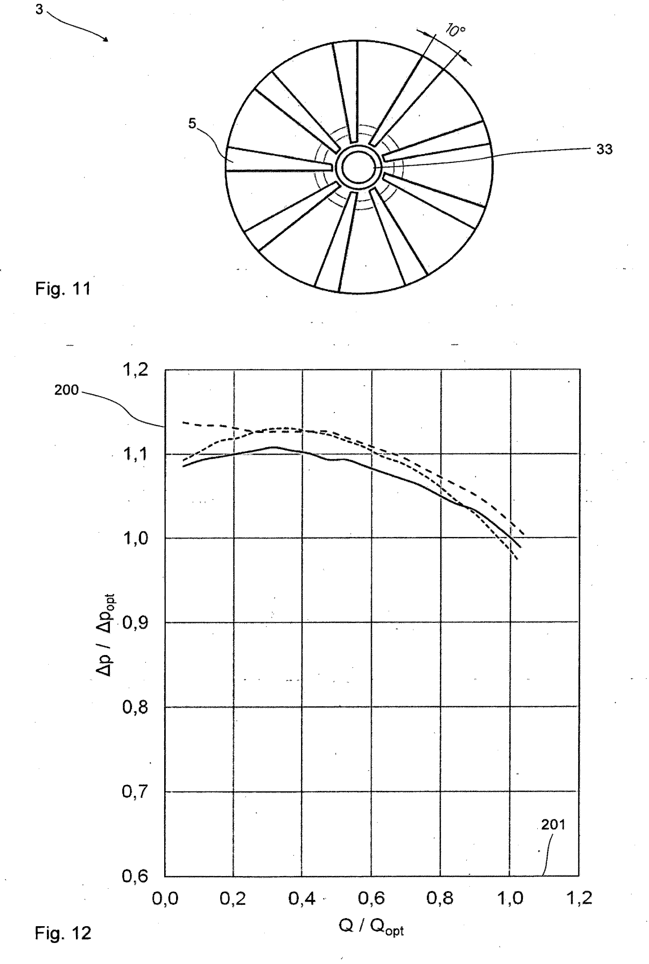

Die Figur 11 zeigt ein Lüfterrad 3, bei dem die Winglets 5 eine Ausdehnung von jeweils 10° des Umfangs des Lüfterrades 3 aufweisen. Für derart dimensionierte Winglets 5 konnten besonders gute strömungstechnische Eigenschaften nachgewiesen werden. Vorteilhafte Strömungseigenschaften konnten auch für Dimensionen zwischen 5° und 15° des Umfangs des Lüfterrades 3 beobachtet werden.

In der Figur 12 sind drei verschiedene Druckverläufe skizziert, bei denen der Druck gegen den Volumenstrom aufgetragen wurde. Dabei sind die Achsen auf einen optimalen Betriebspunkt der Konfiguration ohne Winglets 5 normiert. Der durchgezogene Druckverlauf entspricht dabei einem Lüfterrad 3 ohne Winglets 5. Der grob gestrichelte Druckverlauf wurde bei einem Lüfterrad 3 mit Winglets 5 erfasst,-welche sich in Richtung der Saugseite 24 der Schaufelelemente 4 erstrecken. Der fein gestrichelte Druckverlauf wurde bei einem Lüfterrad 3 erfasst, dessen Winglets 5 sich in Richtung der Druckseite 34 der Schaufelelemente 4 erstrecken. Die Winglets weisen eine Ausdehnung von 10° des Umfangs des Lüfterrades 3 auf.

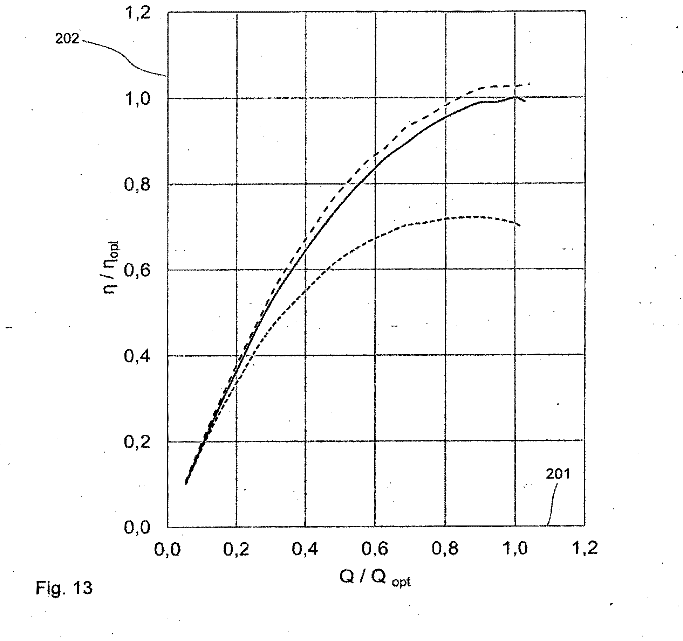

Die Figur 13 zeigt Wirkungsgradverläufe, bei denen der Wirkungsgrad gegen den Volumenstrom aufgetragen wurde. Dabei sind die Achsen auf einen optimalen Betriebspunkt der Konfiguration ohne Winglets 5 normiert. Der durchgezogene Wirkungsgradverlauf entspricht dabei einem Lüfterrad 3 ohne Winglets 5. Der grob gestrichelte Wirkungsgradverlauf wurde bei einem Lüfterrad 3 mit Winglets 5 erfasst, welche sich in Richtung der Saugseite 24 der Schaufelelemente 4 erstrecken. Der fein gestrichelte Wirkungsgradverlauf wurde bei einem Lüfterrad 3 erfasst, dessen Winglets 5 sich in Richtung der Druckseite 34 der Schaufelelemente 4 erstrecken. Die Winglets weisen eine Ausdehnung von 10° des Umfangs des Lüfterrades 3 auf.

### Bezugszeichenliste:

- 1: Atemtherapiegerät
- 2: Lüftereinrichtung
- 3: Lüfterrad
- 4: Schaufelelement
- 5: Winglet
- 6: Scheibe
- 13: Seite
- 14: Längsseite
- 16: Tragscheibe
- 23: Seite
- 24: Saugseite
- 26: Deckscheibe
- 33: Nabe
- 34: Druckseite
- 100: Bedieneinrichtung
- 101: Anzeigeeinrichtung
- 102: Schlauch
- 103: Steuereinrichtung
- 104: Patientenschnittstelle
- 200: Druck
- 201: Volumenstrom
- 202: Wirkungsgrad

## Patentansprüche

1. Atemtherapiegerät (1) mit wenigstens einer Lüftereinrichtung (2) zum Erzeugen einer Atemluftströmung für die Durchführung einer Atemtherapie, wobei die Lüftereinrichtung (2) wenigstens ein drehbares Lüfterrad (3) mit einer Mehrzahl von Schaufelelementen (4) und eine Nabe (33) umfasst,
wobei das Lüfterrad (3) an einer axialen Seite mit einer Tragscheibe (16) ausgestattet ist, wobei die Schaufelelemente an der Tragscheibe (16) und an der Nabe (33) befestigt sind,
**dadurch gekennzeichnet, dass** die Schaufelelemente (4) wenigstens teilweise mit jeweils wenigstens einem an wenigstens einer axialen Längsseite (14) des Schaufelelements (4) wenigstens abschnittsweise verlaufenden Winglet (5) ausgestattet sind.

2. Atemtherapiegerät (1) nach dem vorhergehenden Anspruch, wobei das Winglet (5) wenigstens in Richtung einer Saugseite (24) des Schaufelelements (4) gerichtet ist.

3. Atemtherapiegerät (1) nach einem der vorhergehenden Ansprüche, wobei das Winglet (5) in Richtung einer Druckseite (34) und einer Saugseite (24) des Schaufelelements (4) gerichtet ist.

4. Atemtherapiegerät (1) nach einem der vorhergehenden Ansprüche, wobei das Winglet (5) derart an der Längsseite (14) des Schaufelelements (4) angeordnet ist, dass sich das Winglet (5) in Richtung einer Saugseite (24) in gleicher Ausdehnung wie in Richtung einer Druckseite (34) des Schaufelelements (4) erstreckt.

5. Atemtherapiegerät (1) nach einem der vorhergehenden Ansprüche, wobei das Winglet (5) eine Ausdehnung von 1° bis 20° und vorzugsweise von 5° bis 15° des Umfangs des Lüfterrades (3) aufweist.

6. Atemtherapiegerät (1) nach einem der vorhergehenden Ansprüche, wobei die Winglets 5 sich in Richtung der Druckseite 34 der Schaufelelemente 4 erstrecken und wobei die Winglets eine Ausdehnung von 2 - 20° des Umfangs des Lüfterrades 3 aufweisen.

7. Atemtherapiegerät (1) nach einem der vorhergehenden Ansprüche, wobei eine Ausdehnung des Winglets (5) von radial innen des Lüfterrades (3) nach radial außen des Lüfterrades (3) zunimmt.

8. Atemtherapiegerät (1) nach einem der vorhergehenden Ansprüche, wobei die Winglets 5 gekrümmte Schaufelelemente 4 aufweisen.

9. Atemtherapiegerät (1) nach einem der vorhergehenden Ansprüche, wobei das Lüfterrad (3) an nur einer axialen Seite (13) mit wenigstens einer Scheibe (6) ausgestattet ist und wobei die Scheibe (6) als Tragscheibe (16) zur Befestigung der Schaufelelemente (4) geeignet und ausgebildet ist.

10. Atemtherapiegerät (1) nach dem vorhergehenden Anspruch, wobei die Tragscheibe (16), an derjenigen axialen Seite (13) des Lüfterrades (3) angeordnet ist, welche einer mit den Winglets (5) ausgestatteten axialen Seite (23) des Lüfterrades (3) gegenüberliegt.

11. Atemtherapiegerät (1) nach einem der beiden vorhergehenden Ansprüche, wobei die Schaufelelemente (4) innerhalb eines Umfangs der Scheibe (6) angeordnet sind und/oder wobei die Schaufelelemente (4) nicht über den Umfang der Scheibe (6) hinausstehen.

12. Atemtherapiegerät (1) nach einem der vorhergehenden Ansprüche, wobei die Schaufelelemente (4) wenigstens teilweise gerade oder gekrümmt ausgebildet sind.

13. Atemtherapiegerät (1) nach einem der vorhergehenden Ansprüche, wobei das Lüfterrad (3) mit den Winglets einstückig durch ein Spritzgussverfahren hergestellt ist.

14. Atemtherapiegerät (1) nach einem der vorhergehenden Ansprüche, wobei die Materialstärke der Scheibe 6, der Schaufelelemente 4 und der Winglets 5 entsprechend der mechanischen Belastungen optimiert sind, sodass eine optimale Haltbarkeit resultiert und zugleich eine möglichst geringe Masse des Lüfterrades 3 insgesamt resultiert.

15. Atemtherapiegerät (1) nach einem der vorhergehenden Ansprüche, wobei die Winglets 5 eine geringere Materialstärke aufweisen als die Scheibe 6.

16. Atemtherapiegerät (1) nach einem der vorhergehenden Ansprüche, wobei Beispielsweise die Winglets 5 eine geringere Materialstärke aufweisen als die Schaufelelemente 4.

17. Lüfterrad (3) für eine Lüftereinrichtung (2) eines Atemtherapiegerätes (1), wobei die Lüftereinrichtung (2) zum Erzeugen einer Atemluftströmung für die Durchführung einer Atemtherapie eingerichtet ist, wobei das Lüfterrad (3) eine Mehrzahl von Schaufelelementen (4) und eine Nabe (33) umfasst,
wobei das Lüfterrad (3) an einer axialen Seite mit einer Tragscheibe (16) ausgestattet ist, wobei die Schaufelelemente an der Tragscheibe (16) und an der Nabe (33) befestigt sind,
**dadurch gekennzeichnet, dass** die Schaufelelemente (4) wenigstens teilweise mit jeweils wenigstens einem an wenigstens einer axialen Längsseite (14) des Schaufelelements (4) wenigstens abschnittsweise verlaufenden Winglet (5) ausgestattet sind.

18. Atemtherapiegerät (1) nach Anspruch 1 **dadurch gekennzeichnet, dass** die Winglets (5) sich zumindest teilweise in Richtung der Druckseite (34) der Schaufelelemente (4) erstrecken und wobei die Winglets eine Ausdehnung von 2° bis 20° des Umfangs des Lüfterrades (3) aufweisen, wobei das Lüfterrad (3) an einer axialen Seite (13) mit wenigstens einer Scheibe (6) ausgestattet ist und wobei die Scheibe (6) als Tragscheibe (16) zur Befestigung der Schaufelelemente (4) geeignet und ausgebildet ist und/oder wobei die Schaufelelemente (4) nicht über den Umfang der Scheibe (6) hinausstehen.

## Claims

1. A breathing therapy device (1) with at least one fan apparatus (2) for generating a breathing air flow for performing breathing therapy, wherein the fan apparatus (2) comprises at least one rotatable fan (3) with a plurality of vane elements (4) and a hub (33), wherein the fan (3) is equipped on an axial side with a support disc (16), wherein the vane elements are attached to the support disc (16) and to the hub (33), **characterized in that** at least some of the vane elements (4) are each equipped with at least one winglet (5) running at least in portions on at least one axial longitudinal side (14) of the vane element (4).

2. The breathing therapy device (1) according to the preceding claim, wherein the winglet (5) is directed at least in the direction of a suction side (24) of the vane element (4).

3. The breathing therapy device (1) according to one of the preceding claims, wherein the winglet (5) is directed in the direction of a pressure side (34) and a suction side (24) of the vane element (4).

4. The breathing therapy device (1) according to one of the preceding claims, wherein the winglet (5) is arranged on the longitudinal side (14) of the vane element (4) such that the winglet (5) extends in the direction of a suction side (24) to the same extent as in the direction of a pressure side (34) of the vane element (4).

5. The breathing therapy device (1) according to one of the preceding claims, wherein the winglet (5) has an extent of 1° to 20° and preferably from 5° to 15° of the circumference of the fan (3).

6. The breathing therapy device (1) according to one of the preceding claims, wherein the winglets (5) extend in the direction of the pressure side (34) of the vane elements (4) and wherein the winglets have an extent of 2-20° of the circumference of the fan (3).

7. The breathing therapy device (1) according to one of the preceding claims, wherein an extent of the winglet (5) increases from the radially inner part of the fan (3) to the radially outer part of the fan (3).

8. The breathing therapy device (1) according to one of the preceding claims, wherein the winglets (5) have curved vane elements (4).

9. The breathing therapy device (1) according to one of the preceding claims, wherein the fan (3) is equipped with at least one disc (6) on only one axial side (13) and wherein the disc (6) is suitable and designed as a support disc (16) for attaching the vane elements (4).

10. The breathing therapy device (1) according to one of the preceding claims, wherein the support disc (16) is arranged on that axial side (13) of the fan (3) which is opposite an axial side (23) of the fan (3), which side is equipped with the winglets (5).

11. The breathing therapy device (1) according to one of the two preceding claims, wherein the vane elements (4) are arranged within a circumference of the disc (6) and/or wherein the vane elements (4) do not project beyond the circumference of the disc (6),

12. The breathing therapy device (1) according to one of the preceding claims, wherein the vane elements (4) are designed at least partially straight or curved.

13. The breathing therapy device (1) according to one of the preceding claims, wherein the fan (3) is produced integrally with the winglets using an injection molding method.

14. The breathing therapy device (1) according to one of the preceding claims, wherein the material thickness of the disc (6), of the vane elements (4) and of the winglets (5) is optimized according to the mechanical loads so that an optimal durability results and at the same time an overall mass of the fan (3) that is as low as possible results.

15. The breathing therapy device (1) according to one of the preceding claims, wherein the winglets (5) have a lower material thickness than the disc (6).

16. The breathing therapy device (1) according to one of the preceding claims, wherein, for example, the winglets (5) have a lower material thickness than the vane elements (4).

17. A fan (3) for a fan apparatus (2) of a breathing therapy device (1), wherein the fan apparatus (2) is configured to generate a breathing air flow for performing breathing therapy, wherein the fan (3) comprises a plurality of vane elements (4) and a hub (33), wherein the fan (3) is equipped on an axial side with a support disc (16), wherein the vane elements are attached to the support disc (16) and to the hub (33), **characterized in that** at least some of the vane elements (4) are each equipped with at least one winglet (5) running at least in portions on at least one axial longitudinal side (14) of the vane element (4).

18. The breathing therapy device (1) according to claim 1, **characterized in that** the winglets (5) extend at least partially in the direction of the pressure side (34) of the vane elements (4) and wherein the winglets have an extent of 2° to 20° of the circumference of the fan (3), wherein the fan (3) is equipped on an axial side (13) with at least one disc (6) and wherein the disc (6) is suitable and designed as a support disc (16) for attaching the vane elements (4) and/or wherein the vane elements (4) do not project beyond the circumference of the disc (6).

## Revendications

1. Appareil de thérapie respiratoire (1) doté d'au moins un dispositif ventilateur (2) destiné à produire un flux d'air respiratoire pour la réalisation d'une thérapie respiratoire, dans lequel le dispositif ventilateur (2) comporte au moins une roue de ventilateur (3) rotative dotée d'une pluralité d'éléments de pale (4) et un moyeu (33),
dans lequel la roue de ventilateur (3) est équipée d'un disque de support (16) sur un côté axial, dans lequel les éléments de pale sont fixés au disque de support (16) et au moyeu (33),
**caractérisé en ce que** les éléments de pale (4) sont au moins partiellement équipés respectivement d'au moins une ailette (5) s'étendant au moins par endroits sur au moins un côté longitudinal axial (14) de l'élément de pale (4),

2. Appareil de thérapie respiratoire (1) selon la revendication précédente, dans lequel l'ailette (5) est orientée au moins vers un côté aspiration (24) de l'élément de pale (4),

3. Appareil de thérapie respiratoire (1) selon l'une des revendications précédentes, dans lequel l'ailette (5) est orientée vers un côté pression (34) et un côté aspiration (24) de l'élément de pale (4),

4. Appareil de thérapie respiratoire (1) selon l'une des revendications précédentes, dans lequel l'ailette (5) est disposée de telle façon sur le côté longitudinal (14) de l'élément de pale (4), que l'ailette (5) s'étend vers un côté aspiration (24) selon la même extension que vers un côté pression (34) de l'élément de pale (4),

5. Appareil de thérapie respiratoire (1) selon l'une des revendications précédentes, dans lequel l'ailette (5) présente une extension de 1° à 20° et de préférence de 5° à 15° de la circonférence de la roue de ventilateur (3).

6. Appareil de thérapie respiratoire (1) selon l'une des revendications précédentes, dans lequel les ailettes (5) s'étendent vers le côté pression (34) des éléments de pale (4) et dans lequel les ailettes présentent une extension de 2 à 20° de la circonférence de la roue de ventilateur (3).

7. Appareil de thérapie respiratoire (1) selon l'une des revendications précédentes, dans lequel une extension de l'ailette (5) augmente depuis l'intérieur de la roue de ventilateur (3) vers l'extérieur de la roue de ventilateur (3) dans le sens radial.

8. Appareil de thérapie respiratoire (1) selon l'une des revendications précédentes, dans lequel les ailettes (5) présentent des éléments de pale (4) incurvés.

9. Appareil de thérapie respiratoire (1) selon l'une des revendications précédentes, dans lequel la roue de ventilateur (3) est équipée d'au moins un disque (6) sur un seul côté axial (13) et dans lequel le disque (6) est adapté et conçu en tant que disque de support (16) pour la fixation des éléments de pale (4),

10. Appareil de thérapie respiratoire (1) selon l'une des revendications précédentes, dans lequel le disque de support (16) est disposé sur le côté axial (13) de la roue de ventilateur (3) opposé à un côté axial (23) de la roue de ventilateur (3) équipé des ailettes (5).

11. Appareil de thérapie respiratoire (1) selon l'une des deux revendications précédentes, dans lequel les éléments de pale (4) sont disposés à l'intérieur d'une circonférence du disque (6) et/ou dans lequel les éléments de pale (4) ne dépassent pas de la circonférence du disque (6),

12. Appareil de thérapie respiratoire (1) selon l'une des revendications précédentes, dans lequel les éléments de pale (4) sont conçus au moins partiellement rectilignes ou incurvés.

13. Appareil de thérapie respiratoire (1) selon l'une des revendications précédentes, dans lequel la roue de ventilateur (3) est réalisée d'un seul tenant avec les ailettes à l'aide d'un procédé de moulage par injection,

14. Appareil de thérapie respiratoire (1) selon l'une des revendications précédentes, dans lequel l'épaisseur de matériau du disque (6), des éléments de pale (4) et des ailettes (5) est optimisée en fonction des charges mécaniques, de manière à résulter dans une solidité optimale tout en résultant globalement dans une masse aussi faible que possible de la roue de ventilateur (3),

15. Appareil de thérapie respiratoire (1) selon l'une des revendications précédentes, dans lequel les ailettes (5) présentent une épaisseur de matériau plus faible que le disque (6).

16. Appareil de thérapie respiratoire (1) selon l'une des revendications précédentes, dans lequel les ailettes (5) présentent par exemple une épaisseur de matériau plus faible que les éléments de pale (4),

17. Roue de ventilateur (3) pour un dispositif ventilateur (2) d'un appareil de thérapie respiratoire (1), dans laquelle le dispositif ventilateur (2) est configuré pour produire un flux d'air respiratoire destiné à réaliser une thérapie respiratoire, dans laquelle la roue de ventilateur (3) comporte une pluralité d'éléments de pale (4) et un moyeu (33),
dans laquelle la roue de ventilateur (3) est équipée d'un disque de support (16) sur un côté axial, dans laquelle les éléments de pale sont fixés au disque de support (16) et au moyeu (33),
**caractérisée en ce que** les éléments de pale (4) sont au moins partiellement équipés respectivement d'au moins une ailette (5) s'étendant au moins par endroits sur au moins un côté longitudinal axial (14) de l'élément de pale (4),

18. Appareil de thérapie respiratoire (1) selon la revendication 1, **caractérisé en ce que** les ailettes (5) s'étendent au moins partiellement vers le côté pression (34) des éléments de pale (4) et dans lequel les ailettes présentent une extension de 2° à 20° de la circonférence de la roue de ventilateur (3), dans lequel la roue de ventilateur (3) est équipée d'au moins un disque (6) sur un côté axial (13) et dans lequel le disque (6) est adapté et conçu en tant que disque de support (16) pour la fixation des éléments de pale (4), et/ou dans lequel les éléments de pale (4) ne dépassent pas de la circonférence du disque (6),
